# EUROPÄISCHE PATENTANMELDUNG

(11) **EP 1 972 302 A2**
(43) Veröffentlichungstag der Anmeldung: **24.09.2008**
(21) Anmeldenummer: 08153110.5
(22) Anmeldetag: 20.03.2008
(51) Int. Cl.: A61C 13/15

(54) **Vorrichtung und Gerät zur Lichthärtung von Dentalmaterial, insbesondere zur lichtinduzierten Polymerisation**

(30) Priorität: 20.03.2007 DE 202007004266 U
(71) Anmelder: Wegold Edelmetalle AG, 90530 Wendelstein (DE)
(72) Erfinder: Freisleben, Jürgen, 91126, Schwabach (DE)
(74) Vertreter: Eisenführ, Speiser & Partner

(57) **Zusammenfassung**

Die Erfindung betrifft eine Vorrichtung, ein Gerät und ein System zur Lichthärtung von Dentalmaterial, insbesondere zur lichtinduzierten Polymerisation, mit einer Lichthärtkammer, und einer ersten Strahlungsquelle zur Einstrahlung von Licht einer oder mehrerer ausgewählter Wellenlängen und/oder eines oder mehrerer ausgewählter Wellenlängenbereiche, insbesondere aus dem Wellenlängenbereich von 250 nm bis 550 nm, in die Lichthärtkammer. Die Erfindung ist ferner gekennzeichnet durch eine zweite Strahlungsquelle zur Einstrahlung von Wärmestrahlung und gegebenenfalls sichtbarem Licht in die Lichthärtkammer.

## Beschreibung

Die Erfindung betrifft eine Vorrichtung und ein Gerät zur Lichthärtung von Dentalmaterial, insbesondere zur lichtinduzierten Polymerisation.

Im Dentalbereich finden Dentalmaterialien Anwendung, insbesondere im Bereich der Restauration im Mundbereich und bei der Erstellung von Modellen für Zahnersatz oder Zahnteilersatz, die sich mittels einer Einwirkung von Licht härten lassen, also aus einem fließ- beziehungsweise modellierfähigen Zustand in einen verfestigten Zustand überführen lassen.

Aufgabe der vorliegenden Erfindung ist es, eine Vorrichtung und ein Gerät sowie ein diese Vorrichtung und dieses Gerät umfassendes System vorzustellen, mit denen eine Lichthärtung von unterschiedlichen Dentalmaterialien kosten- und zeiteffektiv erreicht werden kann.

Gelöst wird die Aufgabe durch eine Vorrichtung mit den Merkmalen von Anspruch 1, ein Gerät mit den Merkmalen von Anspruch 17 und ein System mit den Merkmalen von Anspruch 22.

Bevorzugte und vorteilhafte Ausführungsformen der Erfindung sind in den abhängigen Ansprüchen näher definiert.

Die erfindungsgemäße Vorrichtung weist neben einer ersten Strahlungsquelle zur Einstrahlung von Licht, insbesondere aus den Wellenlängenbereich von 250 nm bis 550 nm, in eine Lichthärtkammer, eine zweite Strahlungsquelle zur Einstrahlung von Wärmestrahlung und gegebenenfalls sichtbaren Licht in die Lichthärtkammer auf. Auf diese Weise lassen sich unterschiedliche Wellenlängenbereiche zur Lichthärtung in Kombination mit einer Erwärmung des zu härtenden Dentalmaterials erreichen. Hierbei sind Wärmeeinstrahlung und Einstrahlung von Licht zur Härtung unabhängig voneinander, sodass beispielsweise mit der zweiten Strahlungsquelle eine Erwärmung vorgenommen werden kann, wobei nach Erreichen einer gewünschten Temperatur mit Hilfe der ersten Strahlungsquelle eine Lichthärtung, beispielsweise eine Polymerisation, eingeleitet werden kann.

Das erfindungsgemäße Gerät zur Lichthärtung weist eine erste Lichtquelle zur Einstrahlung von Licht zur Härtung des Dentalmaterials neben einer zweiten Lichtquelle zur Einstrahlung von Licht auf das Dentalmaterial auf, wobei die zweite Lichtquelle Licht aussendet, das nicht zur Lichthärtung des Dentalmaterials beiträgt. Insbesondere bei der manuellen Bearbeitung von Dentalmaterial ergibt sich eine Notwendigkeit nach einer ausreichenden Arbeitsfeldbeleuchtung, also einer Ausleuchtung des zu bearbeitenden Dentalmaterials, ohne dass die die Arbeit unterstützende Beleuchtung selbst auf das Dentalmaterial einwirkt und bei diesem eine Härtung hervorruft.

Bevorzugte Ausgestaltungen sind in den Unteransprüchen angegeben.

Im Folgenden wird die vorliegende Erfindung anhand bevorzugter Ausführungsbeispiele und der beiliegenden Zeichnungen näher erläutert. Hier bei ist:
- Fig. 1: eine schematische Querschnittzeichnung in Seitenansicht einer erfindungsgemäßen Vorrichtung,

- Fig. 2: eine schematische Querschnittzeichnung der Vorrichtung aus Fig. 1 in Draufsicht, und
- Fig. 3: eine schematische Ansicht eines erfindungsgemäßen Gerätes.

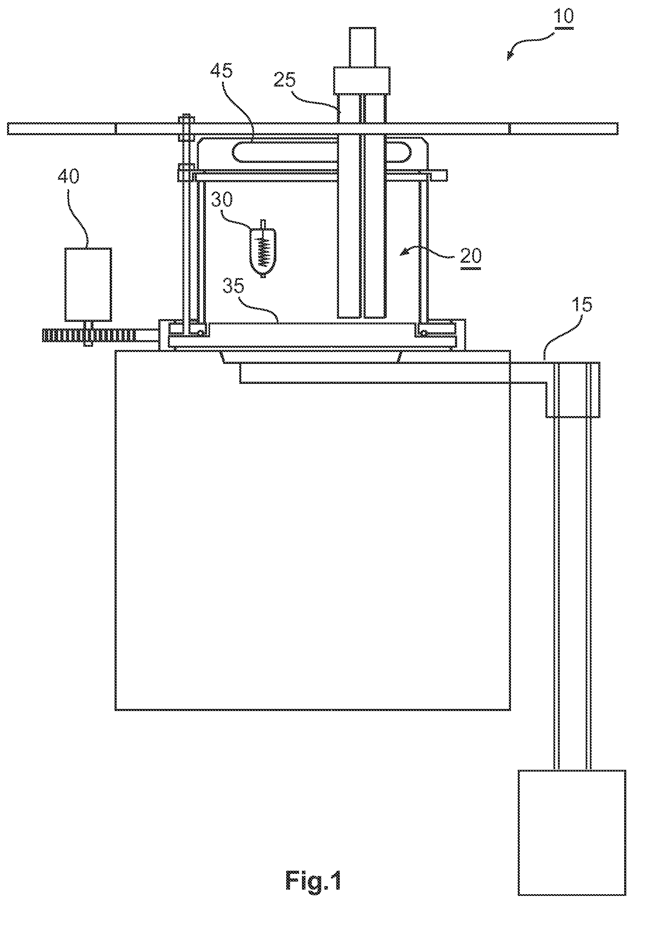

Fig. 1 zeigt eine erfindungsgemäße Vorrichtung 10 mit einer Lichthärtkammer 20, einer ersten Strahlungsquelle 25 und einer zweiten Strahlungsquelle 30, einem drehbar gelagerten Teller 35, einer auf den Teller 35 wirkenden Antriebseinheit 40 und einer dritten Strahlungsquelle 45.

Die Vorrichtung 10 weist einen Lift 15 auf, mit dem der Teller 35 von unten an die Lichthärtkammer 20 herangebracht beziehungsweise von der Lichthärtkammer 20 entfernt werden kann, sodass auf dem Teller befindliches Dentalmaterial in die Lichthärtkammer 20 eingebracht beziehungsweise aus der Lichthärtkammer 20 entfernt werden kann.

In an die Lichthärtkammer 20 herangefahrenem Zustand kann die Antriebseinheit 40 auf den drehbaren Teller 35 einwirken, sodass der Teller während des Betriebs der Vorrichtung 10 in Drehung versetzt werden kann. Der Teller 35 weist auf seiner der Lichthärtkammer 20 zugewandten Oberfläche eine Verspiegelung auf.

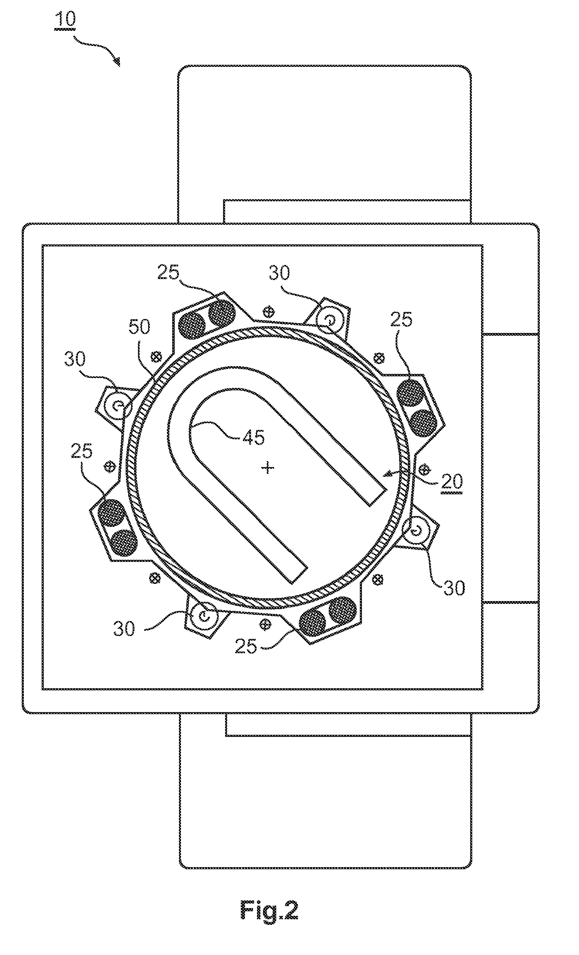

Fig. 2 zeigt die erfindungsgemäße Vorrichtung 10 aus Fig. 1 in einer Querschnittsdraufsicht. Oberhalb der Lichthärtkammer 20, die von einer Trennwand 50 aus Glas umschlossen ist, befindet sich die dritte Strahlungsquelle 45 in Form einer U-förmig gebogenen Xenon-Blitzlampe. Im Bereich außerhalb der Trennwand 50 sind um die Lichthärtkammer 20 herum in regelmäßigem Abstand abwechselnd erste und zweite Strahlungsquellen 25, 30 angeordnet, so dass sich eine vollständige Bestrahlung des Bereichs der Lichthärtkammer 20 erreichen lässt.

Die Vorrichtung 10 weist im Bereich der Lichthärtkammer 20 eine Anzahl von Sensoren (nicht dargestellt) zur Erfassung der Strahlungsflussdichte der ersten, zweiten und dritten Strahlungsquelle 25, 30, 45, die mit einer Selbsttesteinheit (nicht dargestellt) verbunden sind. Die Selbsttesteinheit ist dazu ausgestaltet, mittels eines Vergleiches der mit Hilfe der Sensoren erfassten Strahlungsflussdichten der Strahlungsquellen mit vorbestimmten Sollwerten zu überprüfen, ob gewünschte Betriebsparameter der Vorrichtung jeweilig erreicht werden können. Werden die vorbestimmten Sollwerte nicht erreicht, wird eine Meldung ausgegeben, die den Benutzer darauf hinweist, dass die Leistung der jeweiligen Strahlungsquelle außerhalb des vorgesehenen Bereichs liegt, also beispielsweise zu niedrig für eine zuverlässige Lichthärtung innerhalb einer gewünschten Zeit ist.

Die Vorrichtung führt gemäß eines alternativen Ausführungsbeispiels vor jeder Inbetriebnahme einen Selbsttest durch, geprüft werden hierbei die zur Lichthärtung technisch wichtigsten Komponenten wie die Lampen als Strahlungsquellen (Strahlenflussdichte (Radiometer)), Vakuumpumpe und Heizeinheit. Die Lichthärtkammer ist 12cm im Durchmesser groß und 7cm hoch und sitzt mit großem Abstand über einem Elektronikgehäuse. Ein ausreichend großer, schließbarer Ablageraum für eine Zwischenlagerung von Objekte ist in der Vorrichtung integriert. Ein Lüfter für die zweite und dritte Strahlungsquelle sitzt in der Kammer, der einen Kammerüberdruck erzeugen kann. Bei einem Vakuumbetrieb wird der Lüfter durch ein Ventil von der Lichthärtkammer getrennt.

In der Lichthärtkammer befindet sich ein drehbarer Spiegelteller, der mittels eines Riemenantriebs angetrieben wird. Der Teller ist zwecks Reinigung einfach abnehmbar, ähnlich einem Plattenteller. Der Durchmesser ist flächenfüllend (7-10 cm).

In der Kammer sind Messsensoren in geeignetem Abstand angebracht, die beim Selbsttest die Strahlenflussdichte der Strahlungsquellen. Sofern eine abnehmende Strahlenflussdichte gemessen wird, die sich knapp oberhalb einer Untergrenze befindet, meldet die Vorrichtung "Lampenwechsel erforderlich, Polymerisation ausreichend", stellt dann aber eine Betriebsbereitschaft her. Liegt die Strahlenflussdichte unter der Untergrenze liegen, meldet das Gerät "Achtung : Lampe defekt" und stellt aber erst nach einem Neustart die Bereitschaft her. Die Fehlermeldung bleibt und wird erst bei einer als ausreichend gemessenen Lichtintensität zurückgestellt.

Die Vorrichtung bemerkt selbstständig einen Anschluss einer Vakuumpumpe (Plug & Play). Falls ein solcher Anschluss vorliegt, führt die Vorrichtung ebenfalls einen Test der Vakuumpumpe durch. Bei Nichterreichen eines bestimmten Unterdrucks meldet die Vorrichtung: "Vakuum nicht ausreichend". Die Vorrichtung gibt allerdings nur die Fehlermeldung aus und arbeitet auch bei defekter Vakuumpumpe oder unzureichendem Unterdruck.

Die Strahlungsquellen sind so angebracht, dass sie leicht ausgewechselt werden können. Ein leiser, ausreichend starker Lüfter mit Luftansaugfilter, welcher gut zu reinigen ist, ist an der Rückseite der Vorrichtung angebracht. Bei einem defekten Lüfter oder bei erhöhtem Stromverbrauch wegen eines verstaubten Luftfilters erfolgt eine Fehlermeldung.

Die Innenwandungen der Lichthärtkammer sind lichtreflektierend sein. Die Vorrichtung weist zudem Fassungen für weitere Lampen auf, die eingebaut werden können, um zusätzliche Materialien anderer Hersteller härten zu können.

Der Ablageraum befindet sich zwischen der Polymerisationskammer bzw. Lichthärtkammer und dem Elektronikgehäuse. Er kann mittels einer Klappe oder Tür geöffnet und geschlossen werden und weist Seitenwandungen aus schwarzglänzendem, lichtundurchlässigem Glas auf Der Ablageraum dient zur staub- und lichtfreien Ablage der aktuellen Arbeit bei Unterbrechungen, um später weiter unbeeinträchtigt weiterarbeiten zu können.

Das Elektronikgehäuse ist im unteren Teil des Gerätes angebracht und nimmt die Software und die Elektronik für die Strahlungsquellen auf. Die Bedienung der Vorrichtung findet über ein Foliendisplay statt.

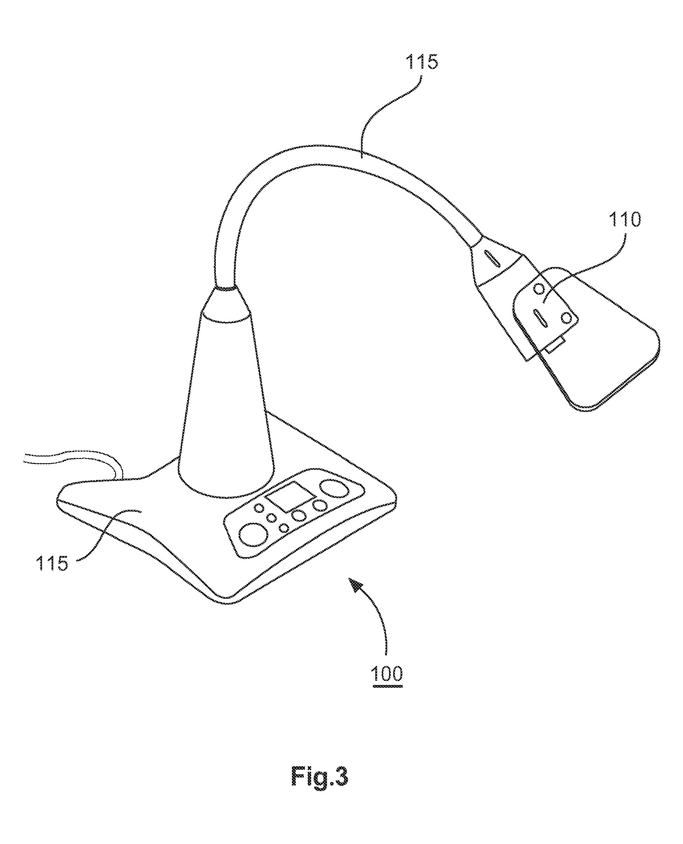

Fig. 3 zeigt ein erfindungsgemäßes Gerät 100 zur Lichthärtung von Dentalmaterial mit einer ersten Lichtquelle und einer zweiten Lichtquelle (nicht dargestellt), die deren Lichtausgänge in einem Kopf 105 angeordnet sind, der über einen Schwanenhals 110 mit einem Standfuß 115 des Gerätes 100 verbunden ist. Das Gerät, das auch als Zwischenhärtungsgerät bezeichnet werden kann, kann vornehmlich zur Zwischenhärtung, also zur schnellen Fixation des Werkstoffes, dienen. Das Gerät ist mit einem Standfuß 115 ausgerüstet, in dem sich eine Photozelle befindet, die bei Abdunkelung durch die Hand, die die erste Lichtquelle aktiviert. Weiterhin befindet sich im Gerät eine Stromversorgung, die das Gerät mit Spannung versorgt. Das Geliergerät kann individuell einstellbare Programme aufweisen. Nach dem Schalten der Lichtschranke beginnt einer digitaler Countdownzähler mit dem Zählen der noch verbleibenden Lichthärtzeit. Wird das Objekt aus der Lichtschranke entfernt, erlischt die erste Lichtquelle und der Counter springt auf die zuvor eingestellte Zeit zurück. Die zweite Lichtquelle weist eine blendfreie LED-Beleuchtung auf, die als Arbeitslicht während des Schichtens dienen kann. Die LED arbeitet unabhängig von der Lichtschranke und ist separat ein- und ausschaltbar. Die Wellenlänge der LED beeinflusst den Aushärtevorgang des lichthärtenden Dentalmaterials nicht.

## Patentansprüche

1. Vorrichtung zur Lichthärtung von Dentalmaterial, insbesondere zur lichtinduzierten Polymerisation, mit:
- einer Lichthärtkammer, und
- einer ersten Strahlungsquelle zur Einstrahlung von Licht einer oder mehrerer ausgewählter Wellenlängen und/oder eines oder mehrerer ausgewählter Wellenlängenbereiche, insbesondere aus dem Wellenlängenbereich von 250 nm bis 550 nm, in die Lichthärtkammer,
**gekennzeichnet durch** eine zweite Strahlungsquelle zur Einstrahlung von Wärmestrahlung und gegebenenfalls sichtbarem Licht in die Lichthärtkammer.

2. Vorrichtung nach Anspruch 1,
**gekennzeichnet durch** einen drehbar gelagerten Teller, der vorzugsweise reflektierend, insbesondere verspiegelt, ist und ferner vorzugsweise entfernbar ist.

3. Vorrichtung nach Anspruch 2,
**gekennzeichnet durch** eine Antriebseinheit zur Drehung des Tellers während einer Lichthärtung, insbesondere mit einem Riemenantrieb.

4. Vorrichtung nach einem der vorstehenden Ansprüche,
**dadurch gekennzeichnet, dass** die erste Strahlungsquelle eine UV-Lampe, insbesondere eine Gasentladungslampe, und/oder eine Leuchtdiode aufweist.

5. Vorrichtung nach einem der vorstehenden Ansprüche,
**dadurch gekennzeichnet, dass** die zweite Strahlungsquelle eine Glühlampe, insbesondere eine Halogenglühlampe, aufweist.

6. Vorrichtung nach einem der vorstehenden Ansprüche,
**dadurch gekennzeichnet, dass** die zweite Strahlungsquelle zur Erwärmung des Inneren der Lichthärtkammer auf 150°C ausgestaltet ist.

7. Vorrichtung nach einem der vorstehenden Ansprüche,
**dadurch gekennzeichnet, dass** die erste und die zweite Strahlungsquelle eine Vielzahl einzelner Strahlungsquelleneinheiten aufweisen, vorzugsweise jeweils 4 Strahlungsquelleneinheiten,
wobei die Lichthärtkammer eine Wandung, eine Oberseite und eine Unterseite aufweist und insbesondere einen kreisförmigen Querschnitt aufweist,
wobei die Strahlungsquelleneinheiten der ersten und der zweiten Strahlungsquelle im Wechsel im Bereich der Wandung um die Lichthärtkammer angeordnet sind, insbesondere in gleichmäßigen Abständen.

8. Vorrichtung nach einem der vorstehenden Ansprüche,
**gekennzeichnet durch** eine dritten Strahlungsquelle zur Einstrahlung von Licht einer oder mehren ausgewählter Wellenlängen und/oder eines oder mehrerer ausgewählter Wellenlängenbereiche, insbesondere aus dem Wellenlängenbereich von 250 nm bis 550 nm, in die Lichthärtkammer,
wobei die erste Strahlungsquelle zur kontinuierlichen Einstrahlung ausgestaltet ist und die dritte Strahlungsquelle zur Erzeugung eines Strahlungspulses ausgestaltet ist.

9. Vorrichtung nach Anspruch 8,
**dadurch gekennzeichnet, dass** die dritte Strahlungsquelle eine Blitzröhre, insbesondere eine Xenon-Blitzröhre, aufweist.

10. Vorrichtung nach einem der Ansprüche 8 oder 9,
wobei die Lichthärtkammer eine Wandung, eine Oberseite und eine Unterseite aufweist und insbesondere einen kreisförmigen Querschnitt aufweist,
**dadurch gekennzeichnet, dass** die dritte Strahlungsquelle im Bereich der Oberseite der Lichthärtkammer angeordnet ist.

11. Vorrichtung nach einem der vorstehenden Ansprüche,
**dadurch gekennzeichnet, dass** die Lichthärtkammer durch eine Trennwand von der ersten, der zweiten und/oder der dritten Strahlungsquelle getrennt ist, wobei die Trennwand für die Strahlung der jeweiligen Strahlungsquelle oder einen ausgewählten Teil davon transparent ist.

12. Vorrichtung nach einem der vorstehenden Ansprüche,
**dadurch gekennzeichnet, dass** die Innenseite der Lichthärtkammer teilweise oder vollständig reflektierend, insbesondere verspiegelt, ausgestaltet ist.

13. Vorrichtung nach einem der vorstehenden Ansprüche,
**gekennzeichnet durch** einen Sensor zur Erfassung der Strahlungsflussdichte der ersten, zweiten und/oder dritten Strahlungsquelle.

14. Vorrichtung nach Anspruch 13,
**gekennzeichnet durch** eine Selbsttesteinheit zum Vergleich der erfassten Strahlungsflussdichte mit einem vorbestimmten Sollwert bei Inbetriebnahme und/oder zu vorbestimmten Zeitpunkten und zum Ausgeben einer Meldung, sofern der vorbestimmte Sollwert nicht erreicht wird.

15. Vorrichtung nach einem der vorstehenden Ansprüche,
**dadurch gekennzeichnet, dass** die Lichthärtkammer gasdicht ausgestaltet ist und die Vorrichtung ferner eine Pumpe zur Erzeugung eines Unterdrucks in der Lichthärtkammer und/oder eine Druckeinheit zur Erzeugung eines Überdrucks in der Lichthärtkammer aufweist.

16. Vorrichtung nach einem der vorstehenden Ansprüche,
**gekennzeichnet durch** einen Ablageraum zur Aufnahme insbesondere unvollständig gehärteten Dentalmaterials, wobei der Ablageraum eine Wandung aufweist, die für eine Härtung des Dentalmaterials bewirkende Strahlung undurchlässig ist.

17. Gerät zur Lichthärtung von Dentalmaterial, insbesondere zur lichtinduzierten Polymerisation mit Handführung, mit einer ersten Lichtquelle zur Einstrahlung von Licht einer oder mehrerer ausgewählter Wellenlängen und/oder eines oder mehrerer ausgewählter Wellenlängenbereiche, insbesondere aus dem Wellenlängenbereich von 250 nm bis 550 nm, auf das Dentalmaterial zur Lichthärtung,
**gekennzeichnet durch** eine zweite Lichtquelle zur Einstrahlung von Licht auf das Dentalmaterial zur Beleuchtung des Dentalmaterials, wobei die zweite Lichtquelle Licht nur solcher Wellenlänge einstrahlt, das nicht zur Lichthärtung des Dentalmaterials beiträgt.

18. Gerät nach Anspruch 17,
**gekennzeichnet durch** einen Standfuß und einen Kopf zur Freisetzung des Lichts der ersten und zweiten Lichtquelle, wobei der Standfuß und der Kopf über einen Schwanenhals miteinander verbunden sind.

19. Gerät nach Anspruch 17 oder 18,
**dadurch gekennzeichnet, dass** die zweite Lichtquelle eine Leuchtdiode aufweist.

20. Gerät nach einem der Ansprüche 17 bis 19,
**gekennzeichnet durch** eine Schalteinheit zur Aktivierung und Deaktivierung der ersten Lichtquelle getrennt von der zweiten Lichtquelle.

21. Gerät nach Anspruch 20,
**dadurch gekennzeichnet, dass** die Schalteinheit zur Aktivierung der ersten Lichtquelle auf eine Benutzereingabe hin, insbesondere auf ein Unterbrechen einer Lichtschranke durch einen Benutzer, und zur Deaktivierung der ersten Lichtquelle nach Ablauf einer vorbestimmten Zeit und/oder nach Ende des Unterbrechens der Lichtschranke ausgestaltet ist.

22. System zur Lichthärtung von Dentalmaterial, insbesondere zur lichtinduzierten Polymerisation, mit einer Vorrichtung nach einem der Ansprüche 1 bis 16 und einem Gerät nach einem der Ansprüche 17 bis 21.
